# EUROPEAN PATENT SPECIFICATION

(11) **EP 0 688 548 B1**
(45) Date of publication and mention of the grant of the patent: **19.04.2000**
(21) Application number: 94109595.2
(22) Date of filing: 22.06.1994
(51) Int. Cl.: A61F 13/15

(54) **Crimping of a fibrous web to a tissue**
Verbinden einer Faserbahn mit einem Gewebe durch Prägen
Fixer une bande fibreuse sur un tissu par gaufrage

(43) Date of publication of application: 27.12.1995
(73) Proprietor: THE PROCTER & GAMBLE COMPANY, Cincinnati, Ohio 45202 (US)
(72) Inventor: Schmitt, John Christian, D-53881 Euskirchen-Kirschheim (DE); Theisgen, Mario Matthias, D-53894 Mechernich/Eiserfey (DE)
(74) Representative: Hirsch, Uwe Thomas

(56) References cited:
- EP-A- 0 354 196
- WO-A-80/01455
- FR-A- 2 274 241
- US-A- 2 118 169
- US-A- 2 916 037
- US-A- 3 559 650

## Description

### Field of the invention

The present invention relates to absorbent articles having absorbent cores comprising multiple absorbent layers which are joined to each other. In particular the present invention relates to absorbent structures which have one of the absorbent layers being shorter in manufacturing direction of the absorbent articles than at least one of the other layers and which are joined to at least one of the other layers by crimping. The present invention also relates to a process of crimping of a central patch of a fibrous web to a tissue to replace previously necessary adhesive and the respective process to make such absorbent structures or embodiments of the present invention.

### Background of the invention

US-A-3,661,680 discloses a process for making an absorbent core comprising a layered construction of tissue, airfield and tissue. The process is indicated to combine the absorbent core by pressure rolls, however, by crimping along selected longitudinal lines. No cross-linked fibers and no super absorbent polymers are mentioned.

EP-A-593 relates to pad integrity improvements by use of a selected tissue which is placed between fibrous pads. Therefore this is the closest prior art for the basic core construction according to the present invention. However only compression to densify but no crimping to join the fibrous pads to the tissue is mentioned.

EP-A-21662 relates to a wicking improvement by use of a special pattern of embossing a fibrous core to a tissue. However, no benefit for the core integrity is indicated and the process to make the absorbent core construction does not mention crimping but shows a patterned compression roll.

EP-A-214190 discloses the crimping of multiple layers of tissue to immobilize particles trapped between the layers. The basic principle of longitudinal crimping lines also is disclosed however no cross-linked fibrous patch is mentioned.

EP-A-214867 discloses fibrous structures with embossed wicking lines. This is not related to core integrity but to the placement of the structure within the product. No crimping of tissue to fibrous material is mentioned and no cross-linked fibers are disclosed.

EP-A-399564 discloses wicking improvements due to the selective density of webs of cross-linked cellulose. Densification by calendering is disclosed however no mention of tissue to pad crimping or core integrity improvements are discussed.

EP-A-0 354 196 discloses an absorbent product which comprises an absorbent core as defined in the preamble of claim 1 with two mutually separate material layers. US-A-3 559 650 discloses a flush-disposable sanitary pad. US-A-2 118 169 discloses a diaper pad comprising spaced parallel grooves.

When making absorbent articles it is desirable to have discontinuous patches of absorbent patches disposed on continuos bands of absorbent material, e.g. tissue. This is particularly true for the highly effective but expensive cross linked fibers now starting to be used more and more in the industry. In particular when using high speed machinery the discontinuous patches have to be joined to the continuous bands or else it would not be possible to transport them. This has typically been done by gluing the patches to the tissue.

However, the leading and trailing ends of a patch are problematic to be glued due to on-off characteristics of the glue delivery and application equipment, it also may cause manufacturing efficiency losses due to glue contamination. Therefore patches were often only glued to the tissue in their center leaving the leading and trailing ends without attachment to the tissue. This however then easily leads to flying patches during production, even further reducing productivity. All these problems are even aggravated if the process of making absorbent cores includes a turning point where the band of already combined material is turned upside down, which has been found useful in obtaining particular products.

Therefore the problem underlying the present invention is to provide an absorbent core comprising a first absorbent structure, a tissue and a second fibrous absorbent structure which is shorter than the tissue in one direction and otherwise not extending beyond the periphery of the tissue. The tissue being joined to the second absorbent structure by crimping.

Preferably the crimping is done along one or several discrete lines. Even so not necessary for the present invention it is preferred if the interface between tissue and the second absorbent structure is free of adhesives.

An additional aspect of the present invention is a continuous process for the manufacture of absorbent cores according to the above description, comprising the steps of placing a continuous tissue on a conveyer belt; forming and depositing in discrete sequence a second fibrous absorbent structure on the continuous tissue; joining the tissue and the second fibrous absorbent structure to each other by crimping; forming the first absorbent structure as a continuous band and placing it onto the tissue on the opposite side from where said first fibrous structure is placed and finally severing the continuous band of tissue and first absorbent structure between one and the following of the second absorbent structures.

### Summary of the invention

The present invention relates to an absorbent core extending along a first axis and perpendicular thereto along a second axis and being produced in the direction of the first axis. The absorbent core comprises a first absorbent structure which has the same first and second axis as the absorbent core, a tissue and a second absorbent structure. The tissue has a first and a second surface and has substantially the same length as the first absorbent structure along the first axis.

The second absorbent structure is shorter than the tissue along the first axis and does not extend beyond the periphery of the tissue in direction of the first axis, preferably in any direction.The second absorbent structure usually is a fibrous absorbent structure preferably comprising cross-linked fibers. The first absorbent structure can be selected from a variety of absorbent structures but preferably also comprises fibers in particular in combination with polymer absorbent gelling material most preferably in particulate form. These absorbent gelling materials may also be present in the second absorbent structure.

The first absorbent structure is contiguous with the first surface of the tissue and is co-extensive along the first axis with that tissue. The absorbent core is constructed such that the tissue on its second surface and the second absorbent structure are joined to each other by crimping.

In the preferred embodiments according to the present invention the second absorbent structure does not extend to the periphery of the tissue at all. However the crimping extends along the first axis at least to one end of the second absorbent structure preferably the crimping extends along the first axis to both ends of the second absorbent structure and for simplicity of manufacturing it is most preferred that the crimping is continuous thereby extending along the full length of the tissue along the first axis.

The crimping may form one of several discrete lines along the first axis and preferably is used to replace adhesive on the second surface on the tissue in order to prevent clogging of the surface by adhesive, improve the manufacturing by elimination of glue heating, glue delivery and application equipment and the problems associated with the required on-off cycling of glue applications.

Accordingly the present invention also relates to a continuous process for the manufacture of the absorbent core of the present invention. This process comprises the steps of
- placing a continuous tissue on a conveyor belt;
- forming and depositing in discrete sequence second fibrous absorbent structures on the second side of the tissue;
- joining the tissue and the second absorbent structure to each other by crimping;
- forming the first absorbent structure as a continuous band and placing it on the first side of the tissue;
- severing the continuous band of tissue and first absorbent structure between one and the following of the second absorbent structure.

The absorbent core according to the present invention can be used in all kinds of absorbent products particularly in disposable absorbent diapers for babies, children or adults as well as in incontinence inserts or in sanitary napkins.

### Brief description of the drawings

Figure 1 shows a top plan view of a tissue continuously crimped to a second absorbent fibrous structure.
Figure 2 is a cross sectional view of the embodiment of the invention of Figure 1 along line 2/2.
Figure 3 is a cross sectional view of the embodiment of the invention of Figure 1 along the line 3/3.
Figure 4 is a top plan view of a prior art tissue and second absorbent fibrous structure glued to each other.
Figure 5 is a cross sectional view of the prior art embodiment of Figure 4 along line 5/5.
Figure 6 is a cross sectional view of the prior art embodiment of Figure 4 along line 6/6.

### Detailed description of the invention and the drawings

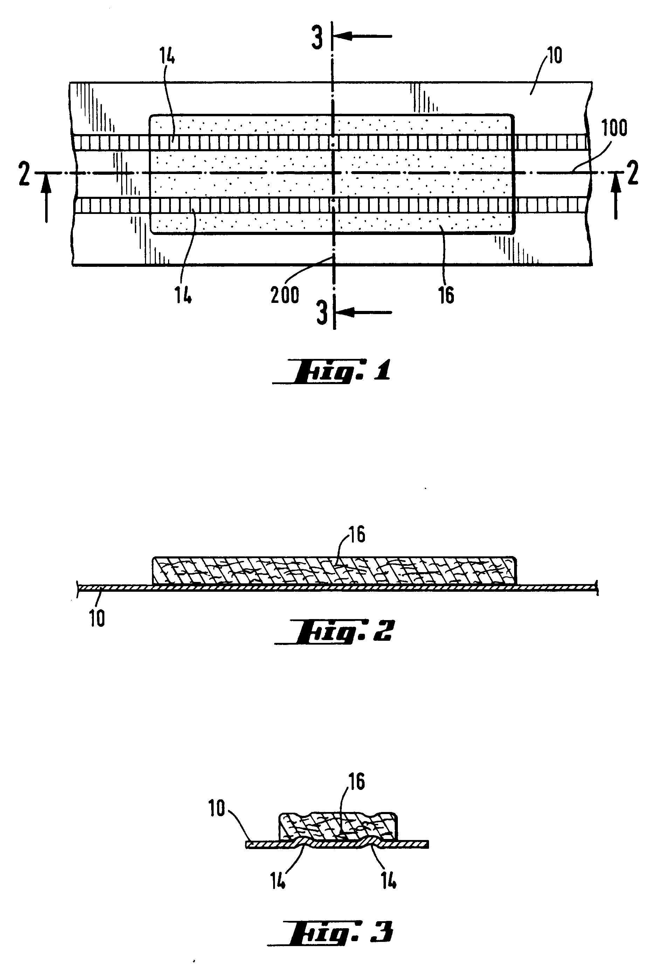

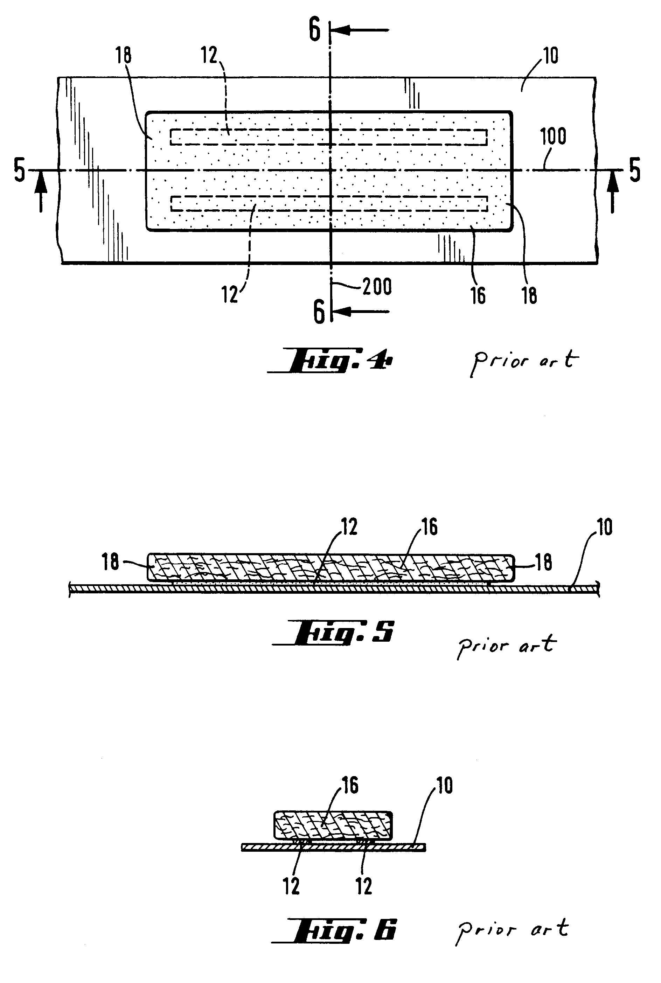

Figure 1 shows a continuous tissue (10) having a first axis (100) and perpendicular thereto a second axis (200). The tissue is overlying a second absorbent structure (16) sharing the axis (100) and (200) with the tissue (10). The tissue (10) and the second absorbent structure (16) are joined by crimping lines (14). The direction of movement during manufacturing is parallel to the first axis (100). When looking at a cross section of this part of the core according to the invention in Figure 2 it can be seen that the second absorbent structure (16) is placed on the second surface of the tissue (10) and joined along the crimping line (14) to that tissue. Not shown in the Figures but part of the absorbent cores of the present invention is a continuous first absorbent structure which is placed on the first side, i.e. the opposite side than the second side, of the tissue (10).

In an alternative cross section along line 3-3 of Figure 1 the crimping lines (14) at which the second absorbent structure (16) and the tissue (10) are compressed together in a crimping process can be seen.

Figure 4 shows a prior art structure also having a first axis (100), a second axis (200) of a tissue (10) and a second absorbent structure (16). However, in this prior art embodiment the joining between the second absorbent structure (16) and the tissue (10) is provided by adhesive lines (12) leaving non-adhered ends (18) between the second absorbent structure (16) and the tissue (10).

As can be seen from Figure 5 the non-adhered ends (18) of the second absorbent structure (16) would easily lift up from the tissue (10) when the tissue is moved in a direction parallel to the first axis (100). Also the width/length of the adhesive lines (12) should be considered since liquid deposited on one side of the absorbent core may have to communicate through the plane where the adhesive prevents such communication across the width and length of the adhesive lines (12).

As used herein the term "disposable absorbent articles" refers to articles which absorb and contain body exudates and more specifically refers to articles which are placed against or in the proximity of the wearer's body to absorb and contain the various exudates discharged from the body of the wearer and which are not intended to be laundered or otherwise restored or reused as an absorbent article (i.e. they are intended to be discarded after a single use and, preferably to be recycled, composted or otherwise disposed of in an environmentally compatible manner). Embodiments of the disposable absorbent article of the present invention are adult incontinence briefs or baby, children's diapers. Examples of the kind of diapers to which the present invention is readily adapted are shown in US patent RE 26,151; US-A-3,860,003; US-A-4,253,461; and US-A-7,704.115.

In general absorbent cores according to the present invention are useful in all disposable absorbent articles.

As used herein the term "absorbent core" refers to all absorbent means in a disposable absorbent article. According to the present invention absorbent cores comprise a first and a second absorbent structure separated by a tissue there between. However further absorbent layers may be comprised in the absorbent cores if deemed appropriate for the particular use of the absorbent article.

In the following reference will be made often to absorbent diapers which however should not mislead in that sanitary napkins, catamenials, panty liners or incontinence inserts represent possible end products in which the absorbent structures according to the present invention may be utilized.

The outside surface of a diaper usually is the surface farthest from the wearer during use of the diaper. The backsheet preferably forms most of the outside surface. The inside surface is that surface of the diaper opposite the outside surface and is preferably formed by the topsheet. Preferably, the inside surface of the diaper is coextensive with the outside surface and in general the inside surface is for the greater part in contact with the wearer when the diaper is used. Typically the outside surface and the inside surface include the absorbent core such that the topsheet and the backsheet extend beyond the core periphery and are joined to each other.

The first and second absorbent structure of the present invention may comprise any absorbent means which are generally compressible, comformable, non-irritating to the wearer's skin, and capable of absorbing and retaining liquids such as urine menses and other body exudates. The absorbent structure may be manufactured in a wide variety of sizes and shapes (e.g., rectangular, hourglass, asymmetric, T-shaped, etc.) and from a wide variety of liquid absorbent materials commonly used in disposable absorbent articles such as communited wood pulp which is generally referred to as airfelt. Examples of other suitable absorbent materials include creped cellulose wadding, meltblown polymers including coform, cross-linked cellulosic fibers, tissue including tissue wraps and tissue laminates, absorbent foams, absorbent sponges, superabsorbent polymers, absorbent gelling materials or any equivalent materials or combination of materials. The configuration and construction of the absorbent core may also be varied e.g., the absorbent core may have varying caliper zones, a hydrophillic gradient, a superabsorbent gradient (as in concentration or particle size for granular superabsorbents), or lower average density and lower average basis weight aquisition zones; or may comprise one or more layers or structures. The total absorbent capacity of the absorbent core should, however, be compatible with the design exudate loading and the intended use of the core.

A preferred embodiment of a diaper has an hourglass-shaped absorbent core. An exemplary absorbent structure readily adaptable for use in the absorbent core of the present invention is described in US Patent 4,610,678 4,673,402 4,888,231. The absorbent core may also be adapted from the commercially successful absorbent member described in US Patent 4,834,735 entitled "High Density Absorbent Members Having Lower Density and Lower Basis Weight Aquisition zones". Other preferred absorbent cores are described in US Patents 4,685,915 and 4,781,710 as comprising fibrous structures having areas of different absorbent capacity, density, or liquid acquisition speed. An alternative thin absorbent core useful in the present invention may be found in US Patent 4,600,458. Another preferred absorbent core design provides for a cross linked cellulose patch essentially without superabsorbent on top of an airfelt with superabsorbent mixture comprising more than 30% superabsorbent.

Preferably the second absorbent structure comprises cross-linked fibers, in particular cross-linked cellulose fibers. In general the first and the second absorbent structures can comprise the same absorbent materials which can be any of those known in the art. The difference between the first and the second absorbent structure according to the invention will be depending on the adaptation of the respective structure to the particular function in the context of the specific absorbent article for which the absorbent core is designed for.

The tissue used between the first and the second absorbent structure according to the invention can be any kind of crimpable tissue usual in the art. Typically tissues comprising wood pulp fibers are used but the tissue fibers may comprise synthetic fibers as well as natural fibers or the tissue may even be a laminate in itself comprising several layers of various tissue type materials or even a laminate structure which comprises particulate materials like for example super absorbent polymers between layers.

The absorbent core is superposed on the backsheet and is preferably joined thereto by a core attachment means.
The backsheet is impervious to liquids (e.g. urine) and is preferably manufactured from a thin plastic film, preferably a thermoplastic film, although other flexible liquid impervious materials may also be used. As used herein, the term "flexible" refers to materials which are compliant and which will readily confirm to the general shape and contours of the human body. The backsheet prevents the exudates absorbed and contained in the absorbent core from soiling articles which contact the diaper such as bedsheets and undergarments.

The backsheet may thus comprise polymeric films such as thermoplastic films of polyethylene or polypropylene, or composite materials such as a film-coated nonwoven material. For economic, aesthetic, and ecological reasons, the backsheet preferably has an average nominal caliper, i.e. calculated caliper, of less than about 0.051 mm, more preferably a calculated caliper of from 0.020 mm to 0.036 mm. Preferably, the backsheet is a flexible polyethylene film. Exemplary films for use as the backsheet of the present invention are manufactured by Tredegar Industries, Inc. of Terre Haute, Indiana, USA or BP-Chemical PlasTec Rotbuchenstrasse 1, D- 8000 MÜNCHEN, Germany.

The topsheet forming the inside surface of the absorbent article is compliant, soft feeling, and non-irritating to the wearer's skin. Further, the topsheet is liquid pervious permitting liquids (e.g. urine) to readily penetrate through its tickness. A suitable topsheet may be manufactured from a wide range of materials, such as porous foam, reticulated foams, apertured films; or woven or nonwoven webs of natural fibers (e.g. wood or cotton fibers), synthetic fibers (e.g. polyester or polypropylene fibers) or from a combination of natural and synthetic fibers. Preferably, it is made of a material that isolates the wearer's skin from liquids retained in the absorbent core.

The diaper preferably further comprises one or several elasticized leg cuffs for providing improved containment of liquids and other body exudates. Each elasticized leg cuff may comprise several different embodiments for reducing the leakage of body exudates in the leg regions. (The leg cuff can be and is sometimes also referred to as leg bands, side flaps, barrier cuffs, or elastic cuffs). US Patent 3,860,003 entitled "Contractable Side Portions for a disposable diaper" describes a disposable which provides a contractible leg opening having a side flap and elastic members to provide an elasticized leg cuff (gasketing cuff). US Patent 4,909,803 entitled "Disposable absorbent article having elasticized flaps" describes a disposable diaper having "stand-up" elasticized flaps (barrier cuffs) to improve the containment of the leg regions. US Patent 4,695,278 entitled "Absorbent article having dual cuffs" describes a disposable diaper having dual cuffs including a gasketing cuff and a barrier cuff. US Patent 4,704,115 entitled "Disposable waist Containment garment" discloses a disposable diaper or incontent garment having side-edge-leakage-guard gutters configured to contain free liquids within the garment.

The diaper may also further comprise an elastic waist feature that provides improved fit and containment or any other features typically provided on diapers or incontinent garments as are known in the art.

The crimping according to the invention is usually conducted by applying high pressure between a hammer and an anvil role which compress the tissue and the second absorbent structure to form a localized high density pattern in which the tissue and the secondary absorbent structure fibers are pressed into each other such that their cohesive and adhesive force allows the second absorbent structure to be joined effectively to the tissue.

There are no particular requirements towards the pattern of the crimping. However since it is known that high density lines in an absorbent article may be beneficial for the liquid distribution in such an absorbent article and crimping lines represent lines of high density they may be formed such as to perform part or all of the liquid distribution function otherwise incorporated by separately formed high density lines in an absorbent core. The crimping lines may also be replaced by other patterns known to be beneficial for liquid distribution, asthetic consideration or otherwise.

For the crimping heated or cold crimping rolls can be used. The primary objective of the crimping is to ensure that the ends of the absorbent second structure are held to the tissue in order to not separate during manufacturing. If propperly made the crimping lines will however continue to hold the secondary absorbent structure to the tissue also during use of the absorbent article and hence improve core integrety without the need for other core adhesion means.

## Claims

1. An absorbent core extending along a first axis (100) and perpendicular thereto along a second axis (200) and being produced in the direction of said first axis (100), said core comprising
- a first absorbent structure having the same first axis (100) and second axis (200) as said absorbent core;
- a tissue (10) having a first and a second surface and substantially the same length as said first absorbent structure along said first axis (100);
- a second absorbent structure (16) having the same first axis (100) and second axis (200) as said absorbent core, being fibrous and being shorter than said tissue (10) along said first axis (100) and not extending beyond the periphery of said tissue (10) in direction of said first axis (100);
said first absorbent structure being contiguous with said first surface of said tissue (10) and co-extensive along said first axis (100) with said tissue (10); and said absorbent core being constructed such that and characterised in that
said tissue (10) on said second surface and said second absorbent structure (16) are joined by crimping.

2. An absorbent core according to claim 1 wherein said second absorbent structure (16) does not extend to the periphery of said tissue (10) in any direction.

3. An absorbent core according to any of the preceding claims wherein said second absorbent structure (16) comprises cross linked fibers.

4. An absorbent core according to any of the preceding claims wherein said first absorbent structure, and preferably said second absorbent structure (16), comprises polymeric absorbent gelling materials.

5. An absorbent core according to any of the preceding claims wherein said crimping extends along said first axis (100) at least to one end of said second absorbent structure (16), preferably said crimping extends through the full length of said tissue (10) along said the first axis (100).

6. An absorbent core according to any of the preceding claims wherein said crimping forms one or several discrete lines along said first axis (100).

7. An absorbent core according to any of the preceding claims wherein said second surface of said tissue (10) is free of adhesive.

8. An absorbent core according to any of the preceding claims wherein said absorbent core is comprised in a disposable absorbent diaper for babies, children or adults, in a incontinence insert or in a sanitary napkin.

9. A continuous process for the manufacture of the absorbent core of claim 1 comprising the steps of
- placing said continuous tissue (10) on a conveyor belt;
- forming and depositing in discrete sequence said second fibrous absorbent structures on said second side of said continuous tissue (10);
- joining said tissue (10) and said second structures by crimping;
- forming said first absorbent structure as a continuous band and placing it on said first side of said tissue (10);
- severing the continuous band of tissue (10) and first absorbent structure between one and the following of said second absorbent structure (16).

10. A continuous process according to claim 9 wherein said crimping is continuous between one and the following of said second fibrous absorbent structures.

## Patentansprüche

1. Ein absorbierender Kern, welcher sich entlang einer ersten Achse (100) und lotrecht dazu entlang einer zweiten Achse (200) erstreckt und welcher in der Richtung der genannten ersten Achse (100) erzeugt wird, wobei der genannte Kern umfaßt
- eine erste absorbierende Struktur mit der gleichen ersten Achse (100) und zweiten Achse (200) wie der genannte absorbierende Kern;
- ein Tissue (10) mit einer ersten und einer zweiten Oberfläche und im wesentlichen der gleichen Länge wie die genannte erste absorbierende Struktur entlang der genannten ersten Achse (100);
- eine zweite absorbierende Struktur (16), welche die gleiche erste Achse (100) und zweite Achse (200) aufweist wie der genannte absorbierende Kern, welche faserig ist und kürzer als das genannte Tissue (10) entlang der genannten ersten Achse (100) ist und sich in der Richtung der genannten ersten Achse (100) nicht über die Peripherie des genannten Tissues (10) hinaus erstreckt;
- wobei die genannte erste absorbierende Struktur mit der genannten ersten Oberfläche des genannten Tissues (10) berührend ist und koextensiv entlang der genannten ersten Achse (100) mit dem genannten Tissue (10) ist;
- und der genannte absorbierende Kern derart aufgebaut ist und dadurch gekennzeichnet ist, daß
das genannte Tissue (10) an der genannten zweiten Oberfläche und die genannte zweite absorbierende Struktur (16) durch Kräuseln verbunden sind.

2. Ein absorbierender Kern nach Anspruch 1, bei welchem die genannte zweite absorbierende Struktur (16) sich nicht in irgendeiner Richtung zur Peripherie des genannten Tissues (10) erstreckt.

3. Ein absorbierender Kern nach einem der vorhergehenden Ansprüche, bei welchem die genannte zweite absorbierende Struktur (16) vernetzte Fasern umfaßt.

4. Ein absorbierender Kern nach einem der vorhergehenden Ansprüche, bei welchem die genannte erste absorbierende Struktur, und vorzugsweise die genannte zweite absorbierende Struktur (16), polymere absorbierende gelbildende Materialien umfaßt.

5. Ein absorbierender Kern nach einem der vorhergehenden Ansprüche, bei welchem die genannte Kräuselung sich entlang der genannten ersten Achse (100) mindestens zu einem Ende der genannten zweiten absorbierenden Struktur (16) erstreckt, wobei vorzugsweise die genannte Kräuselung sich über die volle Länge des genannten Tissues (10) entlang der genannten ersten Achse (100) erstreckt.

6. Ein absorbierender Kern nach einem der vorhergehenden Ansprüche, bei welchem die genannte Kräuselung eine oder verschiedene diskrete Linien entlang der genannten ersten Achse (100) bildet.

7. Ein absorbierender Kern nach einem der vorhergehenden Ansprüche, bei welchem die genannte zweite Oberfläche des genannten Tissues (10) frei von Klebstoff ist.

8. Ein absorbierender Kern nach einem der vorhergehenden Ansprüche, wobei der genannte absorbierende Kern in einer absorbierenden Wegwerfwindel für Babys, Kinder oder Erwachsene, in einer Inkontinenzeinlage oder in einer Hygienevorlage enthalten ist.

9. Ein kontinuierliches Verfahren zur Herstellung des absorbierenden Kerns nach Anspruch 1, welches folgende Schritte umfaßt
- Anordnen des genannten kontinuierlichen Tissues (10) auf einem Förderband;
- Bilden und Ablegen der genannten zweiten faserigen absorbierenden Strukturen in diskreten Sequenzen auf die genannte zweite Seite des genannten kontinuierlichen Tissues (10);
- Verbinden des genannten Tissues (10) und der genannten zweiten Strukturen durch Kräuseln;
- Bilden der genannten ersten absorbierenden Struktur als ein kontinuierliches Band und Anordnung desselben an der genannten ersten Seite des genannten Tissues (10);
- Abtrennen des kontinuierlichen Bandes von Tissue (10) und der ersten absorbierenden Struktur zwischen einer und der folgenden der genannten zweiten absorbierenden Struktur (16).

10. Ein kontinuierliches Verfahren nach Anspruch 9, bei welchem das genannte Kräuseln kontinuierlich zwischen einer und der folgenden der genannten zweiten faserigen absorbierenden Strukturen ist.

## Revendications

1. Ame absorbante s'étendant le long d'un premier axe (100) et perpendiculaire à celui-ci le long d'un deuxième axe (200), et étant produite dans la direction dudit premier axe (100), ladite âme comprenant :
- une première structure absorbante ayant le même premier axe (100) et deuxième axe (200) que ladite âme absorbante ;
- un papier tissu (10) ayant une première surface et une deuxième surface et sensiblement la même longueur que ladite première structure absorbante le long dudit premier axe (100) ;
- une deuxième structure absorbante (16) ayant le même premier axe (100) et deuxième axe (200) que ladite âme absorbante, qui est fibreuse et plus courte que ledit papier tissu (10) le long dudit premier axe (100), et qui ne s'étend pas au-delà de la périphérie dudit papier tissu (10) en direction dudit premier axe (100) ;
ladite première structure absorbante étant contiguë à ladite première surface dudit papier tissu (10) et coïncidant approximativement le long dudit premier axe (100) avec ledit papier tissu (10) ; et ladite âme absorbante est réalisée et caractérisée de manière que :
ledit papier tissu (10) sur ladite deuxième surface et ladite deuxième structure absorbante (16) est réuni par gaufrage.

2. Ame absorbante selon la revendication 1, dans laquelle ladite deuxième structure absorbante (16) ne s'étend pas à la périphérie dudit papier tissu (10), dans aucune direction.

3. Ame absorbante selon l'une quelconque des revendications précédentes, dans laquelle ladite deuxième structure absorbante (16) comprend des fibres réticulées.

4. Ame absorbante selon l'une quelconque des revendications précédentes, dans laquelle ladite première structure absorbante et, de préférence, ladite deuxième structure absorbante (16), comprend des matériaux absorbants gélifiants polymères.

5. Ame absorbante selon l'une quelconque des revendications précédentes, dans laquelle ledit gaufrage s'étend le long dudit premier axe (100), au moins à une extrémité de ladite deuxième structure absorbante (16), de préférence ledit gaufrage s'étend sur la pleine longueur dudit papier tissu (10) le long dudit premier axe (100).

6. Ame absorbante selon l'une quelconque des revendications précédentes, dans laquelle ledit gaufrage forme une ou plusieurs ligne(s) discrète(s) le long dudit premier axe (100).

7. Ame absorbante selon l'une quelconque des revendications précédentes, dans laquelle ladite deuxième surface dudit papier tissu (10) n'a pas d'adhésif.

8. Ame absorbante selon l'une quelconque des revendications précédentes, dans laquelle ladite âme absorbante est comprise dans une couche absorbante jetable pour des bébés, enfants ou adultes, dans un insert pour l'incontinence ou dans une serviette hygiénique.

9. Procédé en continu pour la fabrication de l'âme absorbante selon la revendication 1, comprenant les étapes consistant à :
- placer ledit papier tissu continu (10) sur une courroie transporteuse ;
- former et déposer selon une séquence discrète lesdites deuxièmes structures absorbantes fibreuses sur ledit deuxième côté dudit papier tissu continu (10) ;
- réunir ledit papier tissu (10) et lesdites deuxièmes structures par gaufrage ;
- former ladite première structure absorbante comme une bande continue et la placer sur ledit premier côté dudit papier tissu (10) ;
- couper la bande continue de papier tissu (10) et la première structure absorbante entre une et la suivante desdites deuxièmes structures absorbantes (16).

10. Procédé en continu selon la revendication 9, dans lequel ledit gaufrage est continu entre une et la suivante desdites deuxièmes structures absorbantes fibreuses.
